Europäisches Patentamt
European Patent Office
Office européen des brevets

(11) Publication number: 0 207 036 A1

# (12) EUROPEAN PATENT APPLICATION

(21) Application number: 86850232.9

(22) Date of filing: 19.06.86

(51) Int. Cl.⁴: C07D 211/46 , C07D 401/12 , A61K 31/445

(30) Priority: 20.06.85 SE 8503088

(43) Date of publication of application: 30.12.86 Bulletin 86/52

(84) Designated Contracting States: AT BE CH DE FR GB IT LI LU NL SE

(71) Applicant: Aktiebolaget Hässle
Kärragatan 5
S-431 83 Mölndal(SE)

(72) Inventor: Abrahamsson, Tommy Ingemar
Paradisgatan 16
S-413 16 Göteborg(SE)
Inventor: Gustafsson, Bill Benjamin Rudolf
Krokdalsvägen 106
S-517 00 Bollebygd(SE)

(74) Representative: Näsman, Rune B. G. et al
AB Astra Patent and Trade Mark Department
S-151 85 Södertälje(SE)

## (54) New para-substituted 3-phenoxy-1-piperidinecarbonylaminoalkylamino-propanol-2-s having beta receptor blocking properties.

(57) New compounds of the general formula

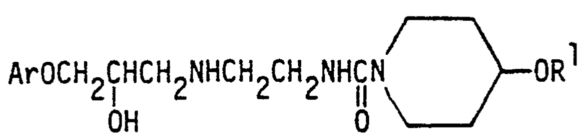

I

wherein R' is hydrogen are described which possess a particularly high and selective affinity for $\beta_1$ receptors. Compounds of formula I wherein R' is an acyl group

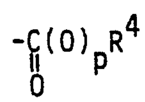

can produce compounds of formula I wherein R' is H by biotransformation.

In formula I Ar is an aromatic or heteroaromatic group, defined by the formula

EP 0 207 036 A1

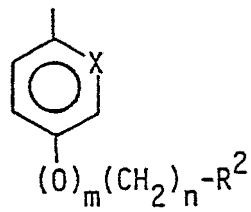

wherein m is an integer 0 or 1, n is an integer 1 to 3 provided that n is an integer 2 to 3 when m is 1 and $R^2$ is a group $-O-R^3$, $-O-(CH_2)_qO-R^3$, a pyrazole group or a group

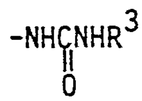

in which groups q is an integer 2 to 3 and $R^3$ is a straight or branched alkyl group having 1-8 carbon atoms or a cycloalkylalkyl group having 4-8 carbon atoms, and X is N or C-R, wherein R is H, CN or $OCH_3$.

In the acyl group

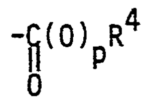

p is an integer 0 or 1 and $R^4$ is an aliphatic, cycloaliphatic or aromatic group, each of said group being optionally substituted or interrupted by heteroatoms.

Further described are methods for preparation of such compounds, pharmaceutical preparations thereof and methods for treatment employing the same.

# New para-substituted 3-phenoxy-1-piperidinecarbonylamino-alkylamino-propanol-2-s having beta receptor blocking properties

## TECHNICAL FIELD

The present invention deals with new β-receptor blocking compounds and the process for their preparation. The new compounds are aimed to be used in the treatment of hypertension, ischemic heart disease, glaucoma and other possible indications where β-adrenoceptor blockers have a beneficial effect.

## BACKGROUND OF THE INVENTION

Since the early 1960:s a vast number of β-receptor blocking compounds have been developed. These compounds are often grouped into two categories of compounds, one where a degree selective activity on $\beta_1$-receptors is shown and one where activity is substantially non-selective between β-receptors. In both categories compounds having a more or less pronounced intrinsic sympatomimetic (β-stimulating) activity are found as well as compounds being substantially void of such stimulating activity. Many of the thus known compounds, and in particular most of the compounds developed into approved pharma ceuticals have the common structure

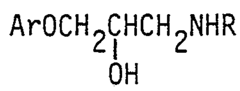

where R is a branched lower alkyl group such as isopropyl or tert. butyl, or occasionally a substituted lower alkyl group and Ar is an aromatic or heteroaromatic group, typically a phenyl group substitued in the 4 and/or 2 position with an aliphatic or heteroaliphatic hydrocarbon that may be further substituted and optionally a halogen atom. Variation in the group Ar is illustrated by reference to known compounds such as alprenolol, atenolol, betaxolol, celiprolol, metoprolol, nadolol, oxprenolol, pafenolol, pindolol, propranolol, sotalol, and timolol.

## DISCLOSURE OF THE INVENTION

The object of the present invention is to obtain potent β-adrenoceptor blockers characterized by a very high selectivity in affinity to peripheral $\beta_1$-adrenoceptors and a long effect duration. The invention includes both compounds with a moderate degree of intrinsic sympatomimetic activity and compounds without any receptor-stimulatory effects.

A highly $\beta_1$ selective blocker would be of benefit causing less degree of $\beta_2$-blockade, and thus unwanted side-effects at higher doses. In some situations such as marked bradycardia and anticipated heart failure, a $\beta_1$ selective blocker with a moderate degree of partial agonist activity is believed to be therapeutically advantageous.

According to the present invention it has been found that compounds of the general formula

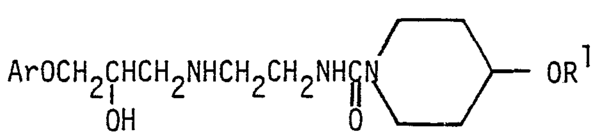

I

wherein $R^1$ is hydrogen possess a particularly high and selective affinity for $\beta_1$ receptors and that compounds of formula I wherein $R^1$ is an acyl group

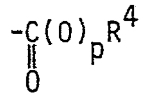

can produce compounds of formula I wherein $R^1$ is H by biotransformation and exert their activity after having been transformed.

The core of the present invention is seen as residing in the provision of the side chain

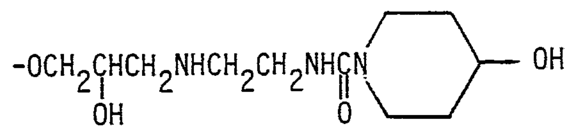

which can be applied to various aromatic or heteroaromatic groups Ar useful in compounds known in the art, and which is optionally esterified at the OH group on the piperidine moiety. Thus, in formula I Ar is an aromatic or heteroaromatic group as known in the art. Notwithstanding the above, subclasses of groups Ar as well as specific groups Ar provide compounds with particularly advantageous properties. In particular Ar is defined by the formula

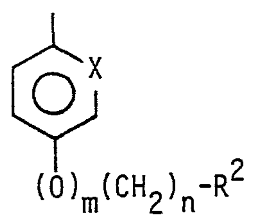

Ia

wherein m is an integer 0 or 1, n is an integer 1 to 3 provided that n is an integer 2 or 3 when m is 1 and $R^2$ is a group $-O-R^3$, $-O-(CH_2)_qO-R^3$ a pyrazole group or a group

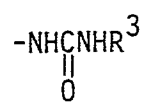

in which groups q is an integer 2 or 3 and $R^3$ is a straight or branched alkyl group having 1-8 carbon atoms or a cycloalkylalkyl group having 4-8 carbon atoms, and X is N or C-R, wherein R is H, CN or $OCH_3$.

In the acyl group

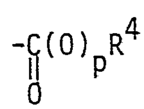

p is an integer 0 or 1 and $R^4$ is an aliphatic, cycloaliphatic or aromatic group such as a straight or branched alkyl group containing 1-20 carbon atoms, a cycloalkyl or a cycloalkylalkyl group containing 3-20 carbon atoms, a phenyl, phenoxy, phenoxymethyl, benzyl or phenylethyl group, each of said groups being optionally substituted, and/or each of said groups being optionally interrupted by heteroatoms.

Preferred compounds of the invention are those compounds of formula I wherein X is CH and in particular those compounds wherein Ar is

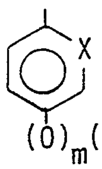

$(O)_m(CH_2)_n-R^2$ and $(O)_m(CH)_n-R^2$ is one of the groups shown below.

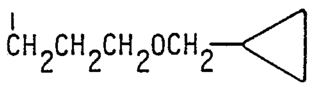

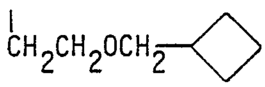

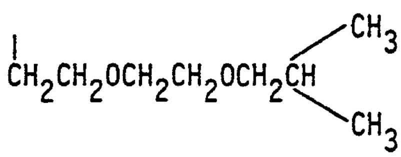

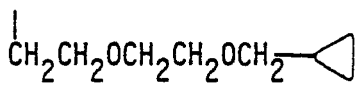

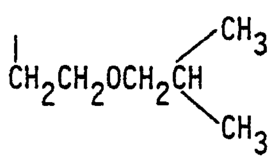

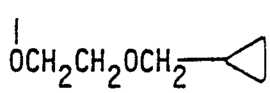

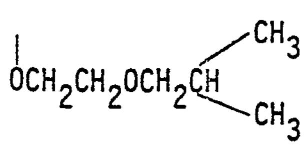

The compounds of formula I wherein $R^1$ is hydrogen have the desired affinity to peripheral $\beta_1$-adrenoceptors. Compounds wherein $R^1$ is an acyl group, as well as other prodrugs or bioprecursors to the compounds where $R^1$ is hydrogen, which may be concieved in further inventions or by applying common knowledge in the art, exert their activity after biotransformation in the living organism to compounds where $R^1$ is hydrogen, and may to some degree possess such activity of their own.

The compounds of the invention may be employed as the bases or pharmaceutically acceptable salts thereof.

Salt forming acids may be used in preparing pharmaceutically acceptable salts of the compounds. These are: hydrohalogen acids, sulfuric acid, phosphoric acid, nitric acid, perchloric acid, aliphatic, alicyclic, aromatic or heterocyclic carboxy or sulfonic acids, such as formic, acetic, propionic, succinic, glycolic, lactic, malic, tartaric, citric, ascorbic, maleic, hydroxymaleic, or pyrovic acid,

phenylacetic, benzoic, p-aminobenzoic, anthranilic, p-hydroxybenzoic, salicyclic or p-aminosalicyclic acid, pamoic acid, methanesulfonic, ethanesulfonic, hydroxyethanesulfonic, ethylenesulfonic, halogenbenzenesulfonic, toluenesulfonic, napthylsulfonic, or sulfanilic acid, methionine, tryptophane, lysine or arginine.

The compounds of the invention may be prepared as outlined below.

For convenience, the part of formula I

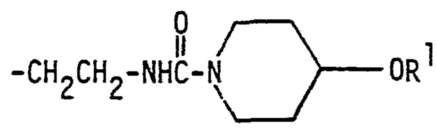

is referred to as $R^N$.

In the groups $R^N$ and Ar, $R^1$, $R^2$, $R^3$, $R^4$ and n are as defined in connection with the specified formulas.

The compounds of the formula I are obtained according to methods such as the following.

a) Reacting a compound of the formula

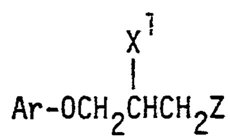

II

or

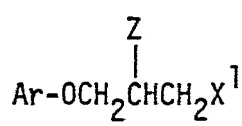

IIa

wherein $X^1$ is a hydroxy group, Z is a hydroxy group or a reactive, esterified hydroxy group, of $X^1$ and Z together form an epoxy group, with a compound of the formula

$H_2NR^N$ III

When Z is a reactive, esterified hydroxy group, it is particularly a hydroxy group esterified with a strong, inorganic acid, preferably a hydrohalogen acid, such as hydrochloric acid, hydrobromic acid, or hydroiodic acid, further sulfuric acid or a strong organic sulfonic acid, e.g. methanesulfonic, trifluoromethanesulfonic, benzenesulfonic, 4-bromobenzenesulfonic or 4-toluenesulfonic acid. Z is preferably chloro, bromo, iodo, methanesulfonyl, trifluoromethanesulfonyl or 4-toluenesulfonyl.

At the use of a reactive ester as a starting material the reaction is preferably carried out in the presence of a basic condensating agent and/or with an excess of an amine. Suitable basic condensating agents are e.g. alkali metal hydroxides such as sodium or potassium hydroxide, alkali metal carbonates such as potassium carbonate, and alkali metal alcoholates such as sodium methylate, potassium ethylate or potassium tert. butylate.

Ring closure to an epoxide may occur as an intermediary reaction in employing a compound of formula II or IIa wherein Z is a reactive esterified hydroxy group. When both Z and $X^1$ are -OH the reaction is carried out in the presence of a metal catalyst, such as Raney nickel. The reaction is preferably carried out in a solvent, which is for example an alkanol having 1 to 4 carbon atoms, by refluxing the reactants in said solvent for a time sufficiently long to give the desired compound, generally 3 to 24 hours.

b) Reacting a compound of the formula

$Ar\text{-}OCH_2CHOHCH_2NH_2$ IV

wherein $R^1$ and $R^2$ are as defined above with a compound of the formula

$ZR^N$ V

wherein Z is as defined above.

The reaction is carried out preferably in the presence of a basic condensating agent and/or excess or an amine. Suitable basic condensating agents are e.g. alkaline alcoholates, preferably sodium or potassium alcoholate, or alkaline carbonates such as sodium or potassium carbonate.

c) Reacting a compound of the formula

ArOH VI

with a compound of formula

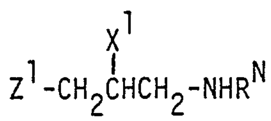

VII

wherein X' is as defined above, and Z' is Z as defined above however Z' is other than hydroxy.

When a reactive ester is used as starting material, the compound of formula VI may suitably be used in the form of its metal phenolate such as an alkali metal phenolate, preferably sodium phenolate. The reaction can also be carried out in the presence of an acid binding agent, preferably a condensating agent, which can form a salt, such as an alkali metal alcoholate of the compound of formula VI.

The reaction is preferably carried out in an autoclave being heated to 80 to 100°C for 1 to 15 hours in an alkanol having 1 to 3 carbon atoms as solvent.

d) Reacting a compound of the formula

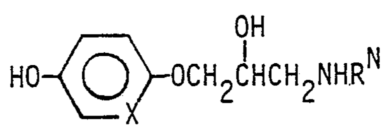

IX

with a compound of the formula

$Z'(CH_2)_n\text{-}R^2$ X

wherein Z' is as defined above, to formation of a compound of formula I wherein Ar is of formula Ia and m is 1.

In the reaction d the reacting hydroxy group is typically activated by a base. The reaction d is preferably carried out in a polar aprotic medium.

e) Reacting a compound of the formula

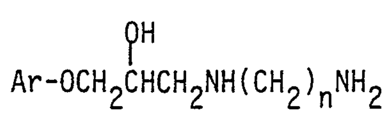

XV

wherein n is as defined above and wherein the nitrogen atom adjacent to the propanol may carry a protective group e.g. a benzyl group, with a compound of the formula

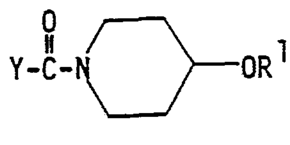

XVI

wherein $R^1$ is as defined above and Y is a leaving group such as halogen, alkoxy, an aryloxy group or an acyloxy group.

f) Splitting off a residue from a compound of formula I above, further substituted with a residue such as a benzyl or substituted benzyl group at a heteroatom.

g) Converting a compound of the formula

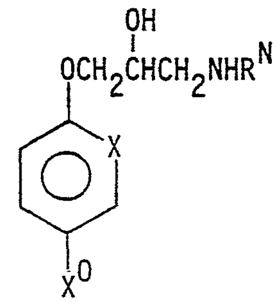

XVII

wherein $X^0$ is a group containing one or more carbon-carbon unsaturation and being convertible to the group $(O)_m(CH_2)_n$-$R^2$ by saturating each such unsaturation therein.

h) Reducing a Schiff's base of the formula

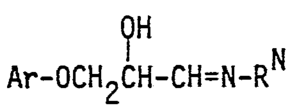

XVIII

This reduction is carried out in a common way, e.g. using a di-light-metalhydride, using a hydride such as a borane with formic acid, or by means of a catalytic hydrogenation, e.g. with hydrogen in the presence of Raney nickel. At the reduction one has to take care that other groups are not affected.

i) Reducing the oxo group to a hydroxy group in a compound of formula

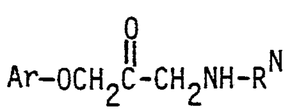

XXI

This reduction is carried out in a common way, especially using a hydride, as mentioned above. The reduction can also be performed in the presence of chiral ligands, thus giving rise to unequal amounts of the enantiomers. The reduction can also be enzymatically performed.

j) Reducing an amidic carbonyl group in a compound of one of the formulas

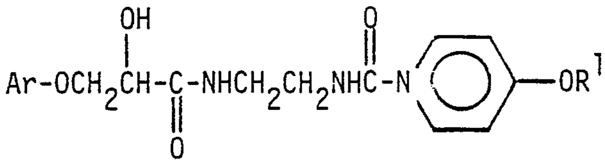

XXII

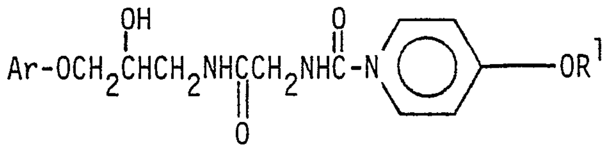

XXIII

The reduction can be carried out according to the above described manner using complex metal hydrides, e.g. lithium aluminium hydride or di-isobutylaluminium hydride. Suitably the reaction takes place in an inert solvent such as an ether, e.g. diethyl ether or tetrahydrofuran. Complex borohydrides can also be used especially when a selective reaction is wanted.

k) To form a compound of formula I where R' is an acyl group

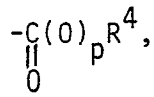

reacting the corresponding compound where R' is H with an acid or acid derivative of the formula

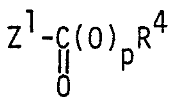

XXIV

wherein Z' is a hydroxy group or a reactive group as defined for a reactive esterified hydroxy group Z above.

l) Reacting a compound of the formula

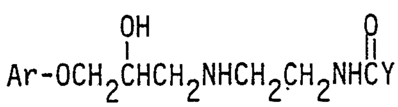

XXV

wherein the nitrogen atom adjacent to the propanol may carry a protective group e.g. a benzyl group, and wherein Y is a leaving group such as halogen, alkoxy, an aryloxy group or an acyloxy group, with a compound of the formula

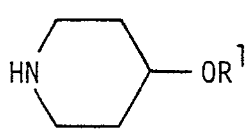

XXVI .

wherein $R^1$ is as defined above.

Depending on the process conditions and the starting material the end product is obtained either in free form or in the form of its acid addition salt, which is included in the scope of the invention. Thus, for example, basic, neutral or mixed salts may be obtained as well as hemiamino, sesqui-or polyhydrates. The acid addition salts of the new compounds may in a manner known per se be transformed into free compounds using e.g. basic agents as alkali or ion exchanger. On the other hand, the free bases obtained may form salts with organic or inorganic acids. In the preparation of acid addition salts preferably such acids are used which form suitable pharmaceutically acceptable salts. Examples of such acids are given above.

These or other salts of the new compounds as e.g. picrates may serve as purifying agents for the free bases obtained as the free bases are transformed into salts, these are separated and the bases are then set free from the salts again. According to the close relationship between the new compounds in free form and in the form of their salts it will be understood from the above and the below that, if possible and unless specified otherwise, reference to the free compound should be taken as a reference to the corresponding salts as well.

As the new compounds of the invention possess at least one asymmetric carbon atom, the invention includes all the possible isomers of the compounds. The new compounds may thus, depending on the choice of starting materials and process, be present as optical antipodes or racemate, or, if they contain at least two asymmetric carbon atoms, be present as an isomer mixture (racemate mixture).

The isomer mixtures (racemate mixtures) obtained may depending on physical-chemical differences of the component, be separated into the both stereoisomeric (diastereomeric) pure racemates e.g. by means of chromatography and/or fractionated crystallization.

The racemates obtained can be separated according to known methods, e.g. by means of recrystallization from an optically active solvent, by means of microorganisms, or by a reaction with optically active acids forming salts of the compound and separating the salts thus obtained, e.g. by means of their different solubility in the diastereomers, from which the antipodes by the influence of a suitable agent may be set free.

Suitably useable optically active acids are e.g. the L-and D-forms of tartaric acid, di-o-tolyl-tartaric acid, malic acid, mandelic acid, camphersulfonic acid or quinic acid. Preferably the more active part of the two antipodes is isolated. Further one of the two enantiomers can be obtained by asymmetrical reduction of the corresponding keto-compound.

Suitably such starting materials are used for the methods presented above, which material leads to groups of end products primarily especially desired and in particular to the specifically described and preferred end products.

The starting materials are known or may, if they should be new, be obtained according to processes known per se.

In clinical use the compounds of the invention are administered normally orally, rectally or by injection in the form of a pharmaceutical preparation, which contains an active component either as free base or as pharmaceutically acceptable, nontoxic acid addition salts, e.g. the hydrochloride, lactate, acetate, sulphamate or the like in combination with a pharmaceutical carrier.

The carrier may be a solid, semisolid or liquid diluent or a capsule. These pharmaceutical preparations are a further object of the invention. Usually the amount of active compound is between 0.1 to 10% by weight of the preparation, suitably between 0.5 to 20% by weight in preparations for injection and between 2 to 50% by weight in preparations for oral administration.

In the preparation of pharmaceutical preparations containing a compound of the present invention in the form of dosage units for oral administration the compound elected may be mixed with a solid, pulverulent carrier, as e.g. with lactose, saccharose, sorbitol, mannitol, starch such as potato starch, corn starch, amylopectin, cellulose derivatives or gelatine, as well as with an antifriction agent as magnesium stearate, calcium stearate, polyethyleneglycol waxes, or the like, and be pressed into tablets. If coated tablets are wanted, the above prepared core may be coated with a concentrated solution of sugar, which solution may contain e.g. gum arabicum, gelatine, talc, titanium dioxide or the like. Furthermore, the tablets may be coated with a lacquer dissolved in an easily volatile organic solvent or mixture of solvents. To this coating a dye may be added in order to easily distinguish between tablets with different active compounds or with different amounts of the active compound present.

In the preparation of soft gelatine capsules - (pearl-shaped, closed capsules), which consist of gelatine and e.g. glycerine or in the preparation of similar closed capsules the active compound is mixed with a vegetable oil. Hard gelatine capsules may contain granules of the active compound in

combination with a solid, pulverulent carrier as lactose, saccharose, sorbitol, mannitol, starch (as e.g. potato starch, corn starch or amylopectin), cellulose derivatives or gelatine.

Dosage units for rectal administration may be prepared in the form of suppositories, which contain the active substance in a mixture with a neutral fat base, or they may be prepared in the form of gelatine-rectal capsules which contain the active substance in a mixture with a vegetable oil or paraffin oil.

Liquid preparations for oral administration may be present in the form of sirups or suspensions, e.g. solutions containing from about 0.2 % by weight to about 20 % by weight of the active substance described, whereby the residue consists of sugar and a mixture of ethanol, water, glycerol and propylene glycol. If desired, such liquid preparations may contain colouring agents, flavouring agents, saccharine and carboxymethyl cellulose as a thickening agent.

Solutions for parenteral administration by injection may be prepared as an aqueous solution of a water-soluble pharmaceutically acceptable salt of the active compound, preferably in a concentration from about 0.1 % by weight to about 10 % by weight. These solutions may also contain stabilizing agents and/or buffering agents and may suitably be available in different dosage unit ampoules.

The preparation of pharmaceutical tablets for peroral use is carried out in accordance with known methods.

The daily dose of the active substance varies and is depending on the type of administration, but as a general rule it is 20 to 500 mg/day at peroral administration and 10 to 200 mg/day at intravenous administration. A recommended dosage for treatment of glaucoma is one drop (50-100 µl) once or twice per day in an affected eye, of a sol ution containing 1-10 mg/ml of active substance.

## BEST MODE OF CARRYING OUT THE INVENTION

The following illustrates the principle and the adaption of the invention, however, without being limited thereto. Temperature is given in degrees Celsius. Structures are shown in the appended tablets 1-3.

Example 1. Preparation of 1-Piperidinecarboxamide, 4-hydroxy-N-(2-((2-hydroxy-3-(4-(3-(cyclopropylmethoxy)propyl)phenoxy)propyl)amino)ethyl)-. (Method a)

12.9 g of the hydrochloride N-2-aminoethyl-4-hydroxypiperidinecarboxamide and 2.3 g of NaOH were stirred in ethanol (100 ml) at 60°C for 1 hour. Thereafter 6.0 g of 3-(4-(3-(cyclopropylmethoxy)-propyl)phenoxy)-1,2-epoxypropane in 50 ml of ethanol was added during 2 hours. The resulting mixture was stirred overnight at 60°C, evaporated to dryness, dissolved in $CH_2Cl_2$ and extracted three times with water, and evaporated to dryness. The residue was chromatographed on 320 g of silica gel 60. The column was first eluted with a $CH_2Cl_2/CH_3OH$ (10:1) mixture and the first 600 ml was discharged. Thereafter fractions of 100 ml were collected. After 18 fractions the methanol content in the mobile phase was increased to 25 %. The fractions 30-36 contained the desired product. After evaporation the residue was recrystallized from acetonitrile. Yield 2.5 g. Melting point 89°C - (base). The structure was confirmed using NMR analysis.

Example 2. Preparation of 1-Piperidinecarboxamide, 4-hydroxy-N-(2-((2-hydroxy-3-(4-(2-(cyclobutylmethoxy)ethyl)phenoxy)propyl)amino)-ethyl)-. (Method a)

12.77 g of the hydrochloride of N-2-aminoethyl-4-hydroxypiperidinecarboxamide and 2.28 g of NaOH were stirred in ethanol (150 ml) for 4 hours. Thereafter 5.1 g of 3-(4-(2-(cyclobutylmethoxy)-ethyl)phenoxy)-1,2-epoxypropane was added. The mixture was stirred at 50°C for 3 days, filtered, evaporated, and chromatographed on silica using methanol/methylene chloride as the mobil phase. At the end pure methanol was used. Yield 2.54 g. Melting point 92°C (base). The structure was confirmed using NMR analysis.

Example 3. Preparation of 1-Piperidinecarboxamide, 4-hydroxy-N-(2-((2-hydroxy-3-(4-(2-(2-(2-methylpropoxy)ethoxy)ethyl)phenoxy)propyl)-amino)ethyl)-. (Method a)

7.6 g of the hydrochloride of N-2-aminoethyl-4-hydroxypiperidinecarboxamide and 1.36 g of NaOH were stirred in ethanol for 1 hour at 60°C. Thereafter 4.0 g of 3-(4-(2-(2-(2-methylpropoxy)ethoxy)-ethyl)phenoxy)-1,2-epoxypropane in 40 ml of ethanol was added during 3 hours. The resulting mixture was stirred at 60°C for 11 hours, evaporated, dissolved in $CH_2Cl_2$, washed three times with water, dried over $N_2SO_4$, filtered and evaporated. The residue was chromatographed on 320 g of silica gel 60. The column was first eluted with 1 l of 20 % $CH_3OH$ in $CH_2Cl_2$ and thereafter with 1 l of 40 % $CH_3OH$ in $CH_2Cl_2$ the following 1.4 l was

collected and evaporated. The residue chrystallized when treated with diisopropyl ether. Yield 2.3 g. Melting point 81 °C (base). The structure was confirmed using NMR analysis.

Example 4. Preparation of 1-Piperidinecarboxamide, 4-hydroxy-N-(2-((2-hydroxy-3-(4-(2-(2-methylpropoxy)ethyl)phenoxy)propyl)amino)ethyl)-. (Method a)

11.2 g of the hydrochloride of N-2-aminoethyl-4-hydroxypiperidinecarboxamide and 2 g of NaOH were stirred in isopropanol (100 ml) for 1 h at 50 °C. Thereafter 5.0 g of 3-(4-(2-(2-methylpropoxy)ethyl)phenoxy)-1,2-epoxypropane in 50 ml of isopropanol was added during 2 hours. The resulting mixture was stirred at 50 °C over night, evaporated, dissolved in 2N hydrochloric acid, and extracted with ether. The aqueous layer was extracted three times with $CH_2Cl_2$, and this organic phase was treated with 10N NaOH, washed with water, dried over $Na_2SO_4$, filtered and evaporated. The residue was chromatographed on 320 g of silica gel 60 according to the procedure of example 3. Yield 1 g. Melting point 94 °C (base). The structure was confirmed using NMR analysis.

Example 5. Preparation of 1-Piperidinecarboxamide, 4-hydroxy-N-(2-((2-hydroxy-3-(4-(2-(cyclopropylmethoxy)ethoxy)phenoxy)propyl)-amino)ethyl)-. (Method 1)

5.2 g of N-(benzyl)-N-(2-((2-hydroxy-3-(4-(2-(cyclopropylmethoxy)ethoxy)phenoxy)propyl)-amino)ethyl)phenoxycarboxamide and 1.4 g of 4-hydroxypiperidine in 75 ml of toluene was stirred at 80 °C for 45 hours. The reaction mixture was extracted twice with diluted NaOH and washed with water, dried over $Na_2SO_4$, filtered and evaporated. The residue was dissolved in 150 ml of ethanol and after adding the catalyst (Pd/C) the mixture was hydrogenated for 2 hours (250 ml $H_2$). The reaction mixture was filtered and evaporated. The residue was chromatographed in the usual way using silica and a $CH_3OH$, $CH_2Cl_2$ mixture. After evaporation of the solvents the residue was crystallized from a isopropyl ether methanol mixture (4:1). Yield 1.42 g. Melting point 86 °C (base). The structure was confirmed using NMR analysis.

Example 6. Preparation of 1-Piperidinecarboxamide, 4-hydroxy-N-(2-((2-hydroxy-3-(4-(2-(2-methylpropoxy)ethoxy)phenoxy)propyl)amino)-ethyl)-. (Method 1)

10.0 g of N-(benzyl)-N-(2-((2-hydroxy-3-(4-(2-(2-methylpropoxy)ethoxy)phenoxy)propyl)amino)-ethyl)phenoxycarboxamide and 2.1 g of 4-hydroxypiperidine in 100 ml of toluene was allowed to react at 100 °C for 8 hours. The mixture was thereafter dissolved in $CH_2Cl_2$ and extracted 2 times with 2M NaOH, washed with water, dried and evaporated. The residue was treated with charchoal, filtered and hydrogenated over 5 % Pd/C in 300 ml ethanol. After consuming 540 ml of $H_2$ gas, the solution was filtered and evaporated. The residue was dissolved in a mixture of isopropanol and ethanol (3:1) and after filtration the product crystallized. The product was filtered off. Yield 6.3 g. Melting point 101 °C (base). The structure was confirmed using NMR analysis.

Example 7. Preparation of 1-Piperidinecarboxamide, 4-hydroxy-N-(2-(((S)-2-hydroxy-3-(4-(2-(cyclopropylmethoxy)ethoxy)phenoxy)propyl)-amino)ethyl)-. (Method a)

1.47 g of the hydrochloride of N-2-aminoethyl-4-hydroxypiperidinecarboxamide was refluxed in 15 ml of ethanol until clear solution. Thereafter 0.45 ml of 15M NaOH solution was added. Thereafter 1.26 g of 3-(4-(2-(cyclopropylmethoxy)ethoxy)phenoxy)-(S)-2-hydroxypropylmethanesulfonate in 15 ml of ethanol was added. The reaction mixture was allowed to react for 2 days at 45 °C. The mixture was thereafter filtered, evaporated and dissolved in $H_2O$ and extracted with $CH_2Cl_2$ at pH 10. The organic layer was thereafter extracted with water at pH 3 - ($H_2SO_4$) and the water phase was extracted with ethyl acetate. The water phase was based to pH 10 and extracted with methylene chloride, washed with water, dried, filtered and evaporated. Yield 0.7 g. Melting point 83 °C. The structure was confirmed using NMR analysis.

Example 8. Preparation of 1-Piperidinecarboxamide, N-(2-((3-(4-(2-amino-2-oxoethyl)phenoxy)-2-hydroxypropyl)amino)ethyl)-4-hydroxy-. (Method a)

8.47 g of the hydrochloride of N-2-aminoethyl-4-hydroxypiperidinecarboxamide and 1.516 g of NaOH were stirred in 200 ml of ethanol for 3 hours. 3.9 g of 3-(4-(carbamoylmethyl)phenoxy)-1,2-epoxypropane was added and the resulting mixture was allowed to react at 50-55 °C for 3 days. The resulting mixture was filtered and evaporated. The residue was chromatographed in the usual way on silica with methanol/methylene chloride as the mobil phase at the end pure methanol was used as the mobile phase. After evaporating of the solvents, the residue was crystallized from ether. Yield 1.0 g. Melting point 105 °C. The structure was confirmed using NMR analysis.

Example 9. Preparation of 2-Methylpropanoic acid, 1-(((2-((2-hydroxy-3-(4-(2-(2-methylpropoxy)ethoxy)-phenoxy)propyl)amino)ethyl)amino)carbonyl)-4-piperidinyl ester. (Method e)

2.08 g of N-benzyl-N-(2-aminoethyl)-3-(4-(2-(2-(2-methylpropoxy)ethoxy)ethyl)phenoxy)-2-hydroxypropylamine, 1.17 g of 4-(2-methyl-propanoyloxy)-piperidinecarbonyl chloride, and 1.7 g of potasium carbonate were allowed to react in 30 ml of methylene chloride for 2 days. The mixture was worked up in the usual way and chromatographed on an Altex column (2.5 x 50 cm) in portions of 1.5 g. The mobile phase was 2.5 % methanol in methylene chloride. 1.26 g of pure product was dissolved in 50 ml of ethanol and hydrogenated (Pd/C). The mixture was thereafter filtered and evaporated. The residue was recrystallized from a mixture of ethyl acetate and isopropanol (1:1). Yield 0.61 g. Melting point 113°C.

Example 10. Preparation of Phenoxyethanoic acid, 1-(((2-((2-hydroxy-3-(4-(2-(2-methylpropoxy)ethoxy)-phenoxy)propyl)amino)ethyl)amino)carbonyl)-4-piperidinyl ester. (Method k)

10 g of N-(Benzyl)-N-(2-((2-hydroxy-3-(4-(2-methylpropoxy)ethoxy)phenoxy)propyl)amino)-ethyl)-4-hydroxypiperidinecarboxamide in 100 ml of $CH_2Cl_2$ and 2.96 ml of pyridine was refluxed and 3.45 g of phenoxyethanoyl chloride in 25 ml of $CH_2Cl_2$ was added during 15 min. The reaction mixture was left under reflux for 1 hour. Thereafter it was washed three times with diluted sulfuric acid and thereafter two times with diluted $Na_2CO_3$-solution. The organic layer was dried, filtered and evaporated. The residue was chromatographed on silica using $CH_2Cl_2$ containing 2-4 % $CH_3OH$ as the mobile phase. Yield 5.2 g. This product was dissolved in ethanol, treated with charcoal, filtered and hydrogenated over Pd/C. The substance consumed 190 ml of $H_2$. The mixture was filtered and evaporated. The residue was thereafter chrystallized from a mixture of isopropanol and ethanol (4:1). Yield 2.6 g. Melting point 87°C. The structure was confirmed using NMR analysis.

Example 11. Preparation of Carbonic acid, 1-(((2-((2-hydroxy-3-(4-(2-(2-methylpropoxy)ethoxy)-phenoxy)propyl)amino)ethyl)amino)carbonyl)-4-piperidinyl phenyl ester. (Method k)

The title compound was prepared in accordance with Ex 10 above using phenyl chloroformate. Yield 0.5 g (from 5.2 g starting material and 1.72 g phenyl chloroformate). Melting point 92°C - (base). The structure was confirmed using NMR analysis.

Example 12. Preparation of Benzenepropanoic acid, 1-(((2-((2-hydroxy-3-(4-(2-(2-methylpropoxy)-ethoxy)phenoxy)propyl)amino)ethyl)amino)-carbonyl)-4-piperidinyl ester. (Method k)

The title compound was prepared in accordance with example 10 above using 6.5 g of the benzyl protected starting material and 2.22 g of 3-phenylpropanoyl chloride as starting materials. Yield 0.5 g. Melting point 87°C (base). The structure was confirmed using NMR analysis.

Example 13. Preparation of Carbonic acid, ethyl 1-(((2-((2-hydroxy-3-(4-(2-(2-methylpropoxy)ethoxy)-phenoxy)propyl)amino)ethyl)amino)carbonyl)-4-piperidinyl ester. (Method k)

The title compound was prepared in accordance with example 10 above using 11.1 g of the benzyl protected starting material and 2.14 ml of ethyl chloroformiat as starting materials. Yield 2.0 g. Melting point 87°C (base). The structure was confirmed using NMR analysis.

Example 14. Preparation of Butanoic acid, 1-(((2-((2-hydroxy-3-(4-(2-(2-methylpropoxy)ethoxy)-phenoxy)propyl)amino)ethyl)amino)carbonyl)-4-piperidinyl ester. (Method k)

The title compound was prepared in accordance with example 10 above using 7.25 g of the benzyl protected starting material and 1.56 g of butanoyl chloride as starting materials. Yield 1.2 g. Melting point 81°C (base). The structure was confirmed using NMR analysis.

Example 15. Preparation of Hexanoic acid, 1-(((2-((2-hydroxy-3-(4-(2-(2-methylpropoxy)ethoxy)-phenoxy)propyl)amino)ethyl)amino)carbonyl)-4-piperidinyl ester. (Method k)

The title compound was prepared in accordance with example 10 above using 4.0 g of the benzyl protected starting material and 1.12 ml of hexanoyl chloride as starting materials. Yield 1.5 g. Melting point 81°C (base). The structure was confirmed using NMR analysis.

Example 16. Preparation of 1-Piperidinecarboxamide, 4-hydroxy-N-(2-((2-hydroxy-3-(4-(2-(2-methylpropoxy)ethyl)-2-methoxyphenoxy)propyl)-amino)ethyl)-. (Method a)

The title compound was prepared in accordance with example 2 above. Reaction time 12 h at 60°C. Yield 3.9 g (52 %) of an oily product. $n_D^{20}$ = 1.5372. The structure was confirmed using NMR analysis.

Example 17. Preparation of 1-Piperidinecarboxamide, 4-hydroxy-N-(2-((2-hydroxy-3-(4-(2-(2-methoxyethoxy)ethyl)phenoxy)propyl)amino)ethyl)-. (Method a)

The title compound was prepared according to example 16 above. The product was chrystallized from diisopropyl ether containing 20 % acetonitrile. Yield 1.5 g (18.8 %). Melting point 76°C (base). The structure was confirmed using NMR analysis.

Example 18. Preparation of 1-Piperidinecarboxamide, 4-hydroxy-N-(2-((2-hydroxy-3-(4-(2-(2-(cyclobutylmethoxy)ethoxy)ethyl)phenoxy)propyl)-amino)ethyl)-. (Method a)

18.6 g of the hydrochloride of N-2-aminoethyl-4-hydroxypiperidinecarboxamide and 3.0 g of NaOH were stirred in ethanol (200 ml) for 3 h at 40°C. Thereafter 8.97 g of 3-(4-(2-(2-(cyclobutylmethoxy)ethoxy)ethyl)phenoxy)-1,2-epoxy-propane in 50 ml of ethanol was added. The mixture was stirred for 3 days at 45°C. Thereafter it was filtered, evaporated, and dissolved in chloroform. The organic layer was thereafter washed trice with water, dried, and evaporated. The residue was recrystallized from ethyl acetate/petroleum spirit 40/60. Yield 5.7 g. Melting point 76°C (base). The structure was confirmed using NMR analysis.

Example 19. Preparation of 1-Piperidinecarboxamide, 4-hydroxy-N-(2-(((R)-2-hydroxy-3-(4-(2-(2-methylpropoxy)ethoxy)phenoxy)propyl)amino)-ethyl)-. (Methods e + f)

2.6 g of N-benzyl-N-(2-aminoethyl)-1-(4-(2-(2-methylpropoxy)ethoxy)phenoxy-3-aminopropan-(R)-2-ol and 1.6 g of 4-benzyloxypiperidinecarbonyl chloride was left standing in 25 ml of methylene chloride and 2 ml of triethyl amine overnight at room temperature. The organic phase was washed with dilute NaOH, dried, filtered, and evaporated. Yield 3.9 g of an oily product, which was dissolved in ethanol and 2M HCl (to pH = 1). Pd/C was added and the mixture was hydrogenated, filtered, and evaporated. The residue was treated with 2M NaOH and extracted with methylene chloride, filtered, and evaporated. The mixture was crystallized from a 1:1 mixture of ethyl acetate and diisopropylether. The crystals were purified by dissolving in 1M HCl, washed twice with ether, made alkaline with 10M NaOH. The chrystals were filtered off, washed with water, and dried. Yield 1.29 g. Melting point 93°C (base). The structure was confirmed using NMR analysis.

Example 20. Preparation of 1-Piperidinecarboxamide, 4-hydroxy-N-(2-(((S)-2-hydroxy-3-(4-(2-(2-methylpropoxy)ethoxy)phenoxy)propyl)amino)-ethyl)-. (Methods e + f)

The title compound was prepared according to example 19 above. The product was crystallized from ethyl acetate. Yield 6.5 g (72 %). Melting point 94°C. The structure was confirmed using NMR analysis.

Example 21. Preparation of 1-Piperidinecarboxamide, 4-hydroxy-N-(2-((2-hydroxy-3-(4-(2-methoxyethyl)phenoxy)propyl)amino)ethyl)-. (Method a)

7.0 g of the hydrochloride of N-2-aminoethyl-4-hydroxypiperidine carboxamide and 1.2 g of NaOH were stirred in ethanol for 2 hours at room temperature. Thereafter 3.1 g of 3-(4-(2-methoxyethyl)-phenoxy)-1,2-epoxypropane in 30 ml of ethanol was added. The mixture was stirred over night at 60°C, filtered, and evaporated. The residue was dissolved in ethyl acetate and extracted with 2M HCl. The aqueous phase was washed with ethyl acetate. Thereafter the aqueous phase was taken to pH 7.5 and extracted with $CHCl_3$. Thereafter the pH was highered to 10, and the aqueous phase again extracted with $CHCl_3$. This procedure was repeated three times. The combined chloroform phase was thereafter evaporated. The residue was recrystallized from toluene. Yield 1.5 g. Melting point 74°C (base). The structure was confirmed using NMR analysis.

Example 22. Preparation of 1-Piperidinecarboxamide, N-(2-((3-(4-(2-(2-(2-(aminocarbonyl)-phenoxy)ethoxy)ethyl)phenoxy)-2-hydroxypropyl)-amino)ethyl)-4-hydroxy-. (Method a)

The title compound was prepared according to example 2 above. Reaction time 6 h at 60°C. The product was crystallized from acetonitrile containing 0.5 % of methanol. Yield 2.0 g (25 %). Melting point 110°C (base). The structure was confirmed using NMR analysis.

Example 23. Preparation of 1-Piperidinecarboxamide, 4-hydroxy-N-(2-((2-hydroxy-3-(4-(2-(2-methylpropoxy)ethyl)-3-cyanophenoxy) propyl)-amino)ethyl)-. (Method a)

The title compound was prepared according to, and on the same scale that example 2 above. Yield 2.7 g of an oily product. $n_D^{20}$ = 1.5545. The structure was confirmed using NMR analysis.

Example 24. Preparation of 2-Methylpropanoic acid, 1-(((2-(((S)-2-hydroxy-3-(4-(2-(2-methylpropoxy)ethoxy)phenoxy)propyl)amino)ethyl)-amino)carbonyl)-4-piperidinyl ester. (Methods e + f)

8.33 g of N-benzyl-N-(2-aminoethyl)-1-(4-(2-methylpropoxy)ethoxy)phenoxy-3-aminopropan-(S)-2-ol and 4.67 g of 4-(1-methylethylcarbonyloxy)-piperidine carbonyl chloride, and 5.53 g of $K_2CO_3$ were stirred in 50 ml of methylene chloride for 2 days at room temperature. The reaction mixture was washed twice with water and evaporated. The residue was chromatographed on 800 g of silica gel. Yield 8.05 g. 3.22 g of this product was dissolved in 125 ml of ethanol and hydrogenated over 10 % Pd/C. 155 ml of hydrogen was consumed. The mixture was filtered, evaporated, and the residue chrystallized twice from ethyl acetate. Yield 1.63 g. Melting point 84°C. The structure was confirmed using NMR analysis.

Example 25. Preparation of 1-Piperidinecarboxamide, 4-hydroxy-N-(2-((2-hydroxy-3-(4-(2-(2-(2-pyridinyloxy)ethoxy)ethyl)phenoxy)propyl)amino)-ethyl)-. (Method a)

The title compound was prepared according to example 18 above. The product was crystallized from acetonitrile. Yield 1.4 g (19.5 %). Melting point 75°C. The structure was confirmed using NMR analysis.

Example 26. Preparation of 1-Piperidinecarboxamide, 4-hydroxy-N-(2-((2-hydroxy-3-(4-(2-(2-(2-cyclopropylmethoxy)ethoxy)ethyl)phenoxy)propyl)-amino)ethyl)-. (Method a)

The title compound was prepared according to example 18 above using 22.4 g of the hydrochloride of N-2-aminoethyl-4-hydroxypiperidinecarboxamide, 4 g of NaOH, and 11.1 g of 3-(4-(2-(cyclopropylmethoxy)ethyl)phenoxy)-1,2-epoxypropane. The product was recrystallized from ethyl acetate. yield 4 g. Melting point 72°C. The structure was confirmed using NMR analysis.

Example 27. Preparation of 1-Piperidinecarboxamide, 4-hydroxy-N-(2-((2-hydroxy-3-(4-((2-(cyclopropylmethoxy)ethoxy)methyl)phenoxy)-propyl)amino)ethyl)-. (Method a)

The title compound was prepared according to example 22 above. The product was crystallized from diisopropyl ether containing 25 % acetonitrile. Yield 1.5 g (15.3 %). Melting point 72°C. The structure was confirmed using NMR analysis.

Example 28. Preparation of 1-Piperidinecarboxamide, 4-hydroxy-N-(2-((2-hydroxy-3-(4-(2-(1H-pyrazol-1-yl)ethoxy)phenoxy)propyl)amino)ethyl)-. (Method a)

The title compound was prepared according to example 2 above. Yield 1.0 g (6 %). Melting point 94°C. The structure was confirmed using NMR analysis.

Example 29. Preparation of 1-Piperidinecarboxamide, 4-hydroxy-N-(2-((2-hydroxy-3-(4-(2-((((1-methyl ethyl)amino)carbonyl)amino)ethyl)phenoxy)-propyl)amino)ethyl)-. (Method a)

The title compound was prepared according to example 2 above. Yield 3 g (43 %), crystallized from ether. Melting point 117°C. The structure was confirmed using NMR analysis.

Example 30. Preparation of cyclohexanecarboxylic acid, 1-(((2-(((S)-2-hydroxy-3-(4-(2-(2-(cyclopropylmethoxy)ethoxy)ethyl)phenoxy)propyl)-amino)ethyl)amino)carbonyl)-4-piperidinyl ester. -(Method e).

The title compound was prepared in accordance with example 9 above using N-benzyl-N-(2-aminoethyl)-3-(4-(2-(2-(cyclopropylmethoxy)-ethoxy)ethyl)phenoxy)-2-(S)-hydroxypropylamine (4.2 g) and 4-cyclohexylcarbonyloxypiperidinecarbonyl chloride (2.1 g) as starting materials. Yield 2.9 g of a yellow oil. $^1$HNMR (500 MHz, $CDCl_3$). δ7.1 2H, 6.8 2H, 5.5 1H, 4.9 1H, 4.1 1H, 3.9 2H, 3.6 8H, 3.3 4H, 3.2 3H, 2.8 7H, 2.3 1H, 1.9 2H, 1.8 2H, 1.7 2H, 1.6 3H, 1.4 2H, 1.25 3H, 1.05 1H, 0.5 2H, 0.2 2H.

Example 31. Preparation of 2,2-dimethylpropionic acid, 1-(((2-(((S)-2-hydroxy-3-(4-(2-(2-(cyclopropylmethoxy)ethoxy)ethyl)phenoxy)propyl)-amino)ethyl)amino)carbonyl)-4-piperidinyl ester. -(Method e).

The title compound was prepared according to Example 9 above using N-benzyl-N-(2-aminoethyl)-3-(4-(2-(2-(cyclopropylmethoxy)ethoxy)ethyl)-phenoxy)-2-(S)-hydroxypropylamine (5.0 g) and 4-(2,2-dimethylpropionyloxy)piperidinecarbonyl chloride (2.8 g) as starting materials. Yield 3.0 g of a yellow oil. $^1$HNMR (500 MHz, $CDCl_3$): δ7.1 2H, 6.8 2H, 5.35 1H, 4.85 1H, 4.05 1H, 3.9 2H, 3.6 6H, 3.5 2H, 3.25 6H, 3.2 6H, 1.8 2H, 1.6 2H, 1.15 9H, 1.05 1H, 0.5 2H, 0.2 2H.

Example 32. Preparation of phenylacetic acid, 1-((-(2-(((S)-2-hydroxy-3-(4-(2-(2-(cyclopropylmethoxy)-ethoxy)ethyl)phenoxy)propyl)amino)ethyl)amino)-carbonyl)-4-piperidinyl ester. (Method e).

The title compound was prepared in accordance with Example 9 above using N-benzyl-N-(2-aminoethyl)-3-(4-(2-(2-(cyclopropylmethoxy)-ethoxy)ethyl)phenoxy)-2-(S)-hydroxypropylamine (4.4 g) and 4-benzylcarbonyloxypiperidinecarbonyl chloride (2.8 g) as starting materials. Yield 2.6 g of a yellow oil. $^1$HNMR ($CDCl_3$, 500 MHz): δ7.3 2H, 7.25 3H, 7.1 2H, 6.8 2H, 5.55 1H, 4.9 1H, 4.1 1H, 3.9 2H, 3.6 8H, 3.5 3H, 3.3 4H, 3.15 2H, 2.8 3H, 2.75 3H, 1.75 2H, 1.55 2H, 1.05 1H, 0.5 2H, 0.2 2H.

Example 33. Preparation of 2,3-dichlorobenzoic acid, 1-(((2-(((S)-2-hydroxy-3-(4-(2-(2-(cyclopropylmethoxy)ethoxy)ethyl)phenoxy)propyl)-amino)ethyl)amino)carbonyl)-4-piperidinyl ester.

2.5 g of N-(2-aminoethyl)-3-(4-(2-(2-(cyclopropylmethoxy)ethoxy)ethyl)phenoxy)-(S)-2-hydroxypropylamine, 25 ml of dry THF and 0.92 g of diisopropylethylamine was mixed and 2.39 g of 4-(2,3-dichlorophenylcarbonyloxy)-piperidinecarbonyl chloride was added in portions during stirring. The mixture was evaporated after 3 h of reaction time. The residue was dissolved in a 1:1 mixture of methylene chloride and ether and washed with water, dried over $Na_2SO_4$, filtered and evaporated to dryness. The synthesis was repeated and the combined residues were chromatographed on $SiO_2$ and $CH_2Cl_2$: MeOH 9:1 as the mobile phase. Yield 0.9 g. $^1$HNMR ($CDCl_3$, 500 MHz, HCl-salt): δ7.6 2H, 7.25 1H, 7.05 2H, 6.8 2H, 6.65 1H, 5.2 1H, 4.55 1H, 4.05 1H, 3.95 1H, 3.7 4H, 3.6 6H, 3.4 3H, 3.3 6H, 2.8 2H, 1.95 2H, 1.8 2H, 1.05 1H, 0.55 2H, 0.2 2H.

Example 34. Preparation of 4-hydroxy-N-(2-(((S)-2-hydroxy-3-(4-(2-(2-(2-cyclopropylmethoxy)ethoxy)-ethyl)phenoxy)propyl)amino)ethyl. (Method 1).

The title compound was prepared in accordance with Example 5 above using N-(benzyl)-N-(2-(((S)-2-hydroxy-3-(4-(2-(cyclopropylmethoxy)-ethoxy)phenoxy)propyl)amino)ethyl)-phenoxycarboxamide (13.0 g) and 4-hydroxypiperidine (3.27 g) as starting materials. Yield 3.1 g. Melting point 77-78°C. The structure was confirmed using NMR analysis.

Example 35. Preparation of hexanoic acid, 1-(((2-((-(S)-2-hydroxy-3-(4-(2-(2-(cyclopropylmethoxy)-ethoxy)ethyl)phenoxy)propyl)amino)ethyl)amino)-carbonyl)-4-piperidinyl ester.

The title compound was prepared according to Example 9 above using N-benzyl-N-(2-aminoethyl)-3-(4-(2-(2-(cyclopropylmethoxy)ethoxy)ethyl)-phenoxy)-2-(S)-hydroxypropylamine (4.5 g) and 4-hexanoyloxypiperidinecarbonyl chloride (2.64 g) as starting materials. Yield 3.1 g. Melting point 25-30°C. $^1$HNMR (500 MHz, $CDCl_3$): δ7.1 2H, 6.8 2H, 5.25 1H, 4.9 1H, 4.1 1H, 3.95 2H, 3.6 8H, 3.35 2H, 3.3 2H, 3.2 2H, 2.85 8H, 2.3 2H, 1.85 2H, 1.6 4H, 1.3 4H, 1.1 1H, 0.9 3H, 0.5 2H, 0.2 2H.

Example 36. Preparation of 2-methylpropanoic acid, 1-(((2-(((S)-2-hydroxy-3-(4-(2-(2-(cyclopropylmethoxy)ethoxy)ethyl)phenoxy)propyl)-amino)ethyl)amino)carbonyl)-4-piperidinyl ester.

The title compound was prepared according to Example 9 above using N-benzyl-N-(2-aminoethyl)-3-(4-(2-(2-(cyclopropylmethoxy)ethoxy)-ethylphenoxy)-2-(S)-hydroxypropylamine (5 g) and 4-(2-methylpropionyloxy)-piperidinecarbonyl chloride (2.6 g) as starting materials. Yield 2.4 g. $^1$HNMR ($CDCl_3$, 500 MHz): δ7.1 2H, 6.8 2H, 6.25 1H, 4.9 1H, 4.0 2H, 3.65 10H, 3.3 4H, 3.15 4H, 2.8 2H, 2.5 1H, 1.85 2H, 1.65 2H, 1.15 6H, 0.9 1H, 0.55 2H, 0.2 2H.

Example 37

An injection solution was prepared by dissolving 1-Piperidinecarboxamide, 4-hydroxy-N-(2-((2-hydroxy-3-(4-(3-(cyclopropylmethoxy)propyl)-phenoxy)propyl)amino)ethyl) • hydrochloride (0.1 g), sodium chloride (0.8 g) and ascorbic acid (0.1 g) in a sufficient amount of distilled water to give 100 ml of solution. This solution, which contains 1 mg of active substance per each ml, was used in filling ampoules, which were sterilized.

Example 38

A sirup containing 2% (weight per volume) of active substance was prepared from the following ingredients:

| | |
|---|---|
| 2-Methylpropanoic acid, 1(((2-((2-hydroxy-3-(4-(2-(2-methylpropoxy)ethoxy)phenoxy)propyl)amino)ethyl)amino)-carbonyl)-4-piperidinyl ester • HCl | 2.0 g |
| Saccharine | 0.6 g |
| Sugar | 30.0 g |
| Glycerine | 5.0 g |
| Flavouring agent | 0.1 g |
| Ethanol 96% | 10.0 ml |
| Distilled water | ad 100.0 ml |

Sugar, saccharine and the ammonium salt were dissolved in 60 g of warm water. After cooling glycerine and a solution of flavouring agents dissolved in ethanol were added. To the mixture water was then added to 100 ml.

Example 39

2-Methylpropanoic acid, 1-(((2-((2-hydroxy-3-(4-(2-(2-methylpropoxy)ethoxy)phenoxy)propyl)-amino)ethyl)amino)carbonyl)-4-piperidinyl ester • hydrochloride (250 g) was mixed with lactose - (175.8 g), potato starch (169.7 g) and colloidal silicic acid (32 g). The mixture was moistened with 10% solution of gelatine and was granulated through a 12-mesh sieve. After drying potato starch (160 g), talc (50 g) and magnesium stearate (5 g) were admixed and the mixture thus obtained was pressed into tablets (10 000) which contain 25 mg of substance. The tablets are sold on the market provided with a breaking score to give another dose than 25 mg or to give multiples thereof when broken.

Example 40

Granules were prepared from 2-methylpropanoic acid, 1-(((2-((2-hydroxy-3-(4-(2-(2-methylpropoxy)ethoxy)phenoxy)propyl)amino)ethyl)-amino)carbonyl)-4-piperidinyl ester • hydrochloride (250 g), lactose (175.9 g) and an alcoholic solution of polyvinylpyrrolidone (25 g). After the drying step the granules were mixed with talc (25 g), potato starch (40 g) and magnesium stearate (2.50 g) and was pressed into 10 000 tablets being biconvex. These tablets are primarily coated with a 10% alcoholic solution of shellac and thereupon with an aqueous solution containing saccharose (45%), gum arabicum (5%), gelatine (4%) and dyestuff (0.2%). Talc and powder sugar were used for powdering after the first five coatings. The coating was then coated with a 66% sugar sirup and polished with a 10% carnauba wax solution in carbon tetrachloride.

Example 41

An eye drop solution may be prepared having the following composition:

| | | |
|---|---|---|
| Active compound (e.g. the compound of Example 26) | | 1-10 mg |
| NaOH 0.1 M or HCl 0.1 M | to pH 7.4 | |
| $Na_2HPO_4$ | | 12.97 mg |
| $NaH_2PO_4 \cdot 2H_2O$ | | 3.57 mg |
| Benzalkonium Chloride | | 0.1 mg |
| Water useful for injection | ad | 1 ml |

Example 42

An eye drop solution may be prepared having the following composition:

| | | | |
|---|---|---|---|
| Active compound (e.g. the compound of Example 26) | | 1-10 | mg |
| NaOH 0.1 M or HCl 0.1 M | to pH | 7.4 | |
| NaCl | | 9 | mg |
| Benzalkonium Chloride | | 0.1 | mg |
| Water useful for injection | ad | 1 | ml |

Table 1

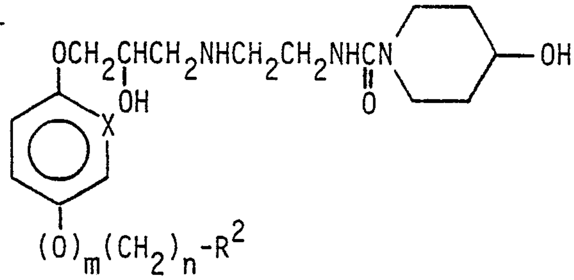

| Example | m | n | $R^2$ | q | $R^3$ | $X^2$ | Isomer[1] |
|---|---|---|---|---|---|---|---|
| 1 | 0 | 3 | $-O-R^3$ | - | $-CH_2$ 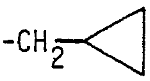 | | |
| 2 | 0 | 2 | $-O-R^3$ | - | $-CH_2$ 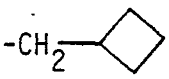 | | |
| 3 | 0 | 2 | $-O-(CH_2)_qO-R^3$ | 2 | $CH_3$<br>$-CH_2CHCH_3$ | | |
| 4 | 0 | 2 | $-O-R^3$ | - | $CH_3$<br>$-CH_2CHCH_3$ | | |
| 5 | 1 | 2 | $-O-R^3$ | - | $-CH_2$ 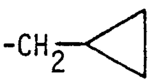 | | |
| 6 | 1 | 2 | $-O-R^3$ | - | $CH_3$<br>$-CH_2CHCH_3$ | | |
| 7 | 1 | 2 | $-O-R^3$ | - | $-CH_2$ 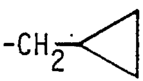 | | S |
| 16 | 0 | 2 | $-O-R^3$ | - | $CH_3$<br>$-CH_2CHCH_3$ | 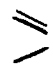 $COCH_3$ | |
| 17 | 0 | 2 | $-O-(CH_2)_qO-R^3$ | 2 | $-CH_3$ | | |
| 18 | 0 | 2 | $-O-(CH_2)_qO-R^3$ | 2 | $-CH_2$ 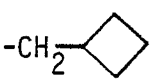 | | |
| 19 | 1 | 2 | $-O-R^3$ | - | $CH_3$<br>$-CH_2CHCH_3$ | | R |
| 20 | 1 | 2 | $-O-R^3$ | - | $CH_3$<br>$-CH_2CHCH_3$ | | S |
| 21 | 0 | 2 | $-O-R^3$ | - | $-CH_3$ | | |
| 22 | 0 | 2 | $-O-(CH_2)_qO-R^3$ | 2 | 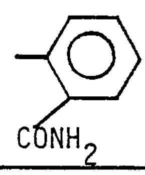 | | |

cont.

[1] Racemate unless stated otherwise

[2] X is 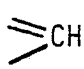 CH unless stated otherwise

Table 1 (cont.)

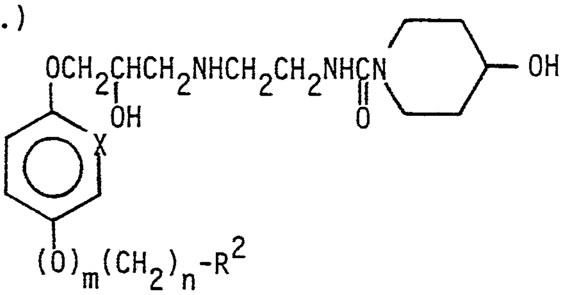

| Example | m | n | $R^2$ | q | $R^3$ | $X^2$ | Isomer[1] |
|---|---|---|---|---|---|---|---|
| 23 | 0 | 2 | $-O-R^3$ | - | $CH_3$<br>$CH_2CHCH_3$ | 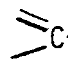C-CN | |
| 25 | 0 | 2 | $-O-(CH_2)_qO-R^3$ | 2 | 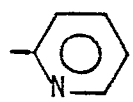 | | |
| 26 | 0 | 2 | $-O-(CH_2)_qO-R^3$ | 2 | $-CH_2$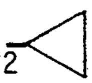 | | |
| 27 | 0 | 1 | $-O-(CH_2)_qO-R^3$ | 2 | $-CH_2$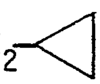 | | |
| 28 | 1 | 2 | 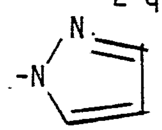 | - | - | | |
| 29 | 0 | 2 | $-NHCNH-R^3$<br>O | - | $CH_3$<br>$-CHCH_3$ | | |
| 34 | 0 | 2 | $-O-(CH_2)_qOR^3$ | 2 | $-CH_2$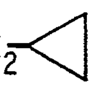 | | S |

[1] Racemate unless stated otherwise

[2] X is 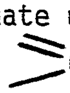CH unless stated otherwise

Table 1 a

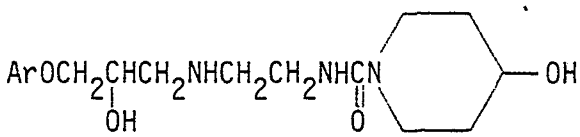

| Example | Ar |
| --- | --- |
| 8 | 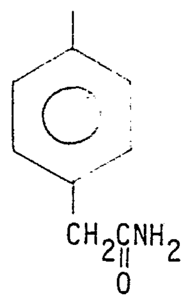 |

Table 2

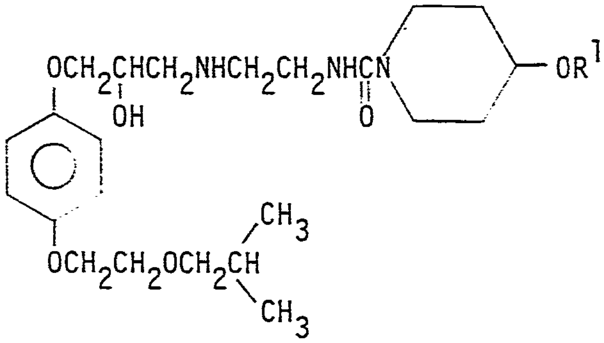

| Example | R[1] | Isomer[1] |
|---|---|---|
| 9 | 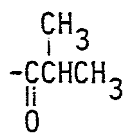 | |
| 10 | 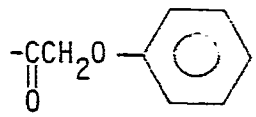 | |
| 11 | 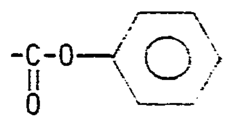 | |
| 12 | 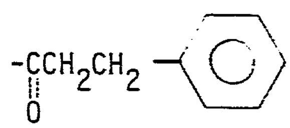 | |
| 13 | 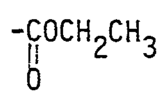 | |
| 14 | 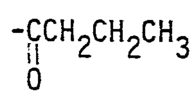 | |

[1] Racemate unless stated otherwise

cont.

| Example | $R^1$ | Isomer[1] |
| --- | --- | --- |
| 15 | 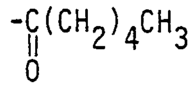 | |
| 24 | 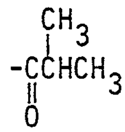 | S |

[1]Racemate unless stated otherwise

Table 3

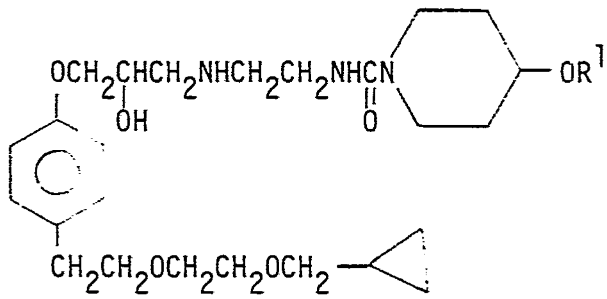

| Example | $R^1$ | Isomer[1] |
|---|---|---|
| 30 | 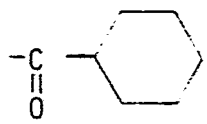 | |
| 31 | 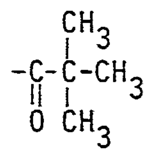 | |
| 32 | 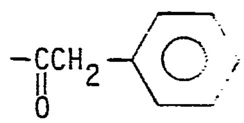 | |
| 33 | 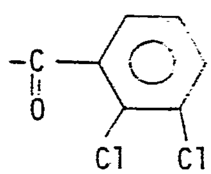 | |
| 35 | 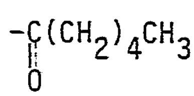 | |
| 36 | 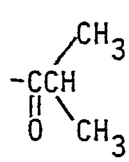 | |

[1]S-form unless stated otherwise

**Claims**

1. A compound of the formula

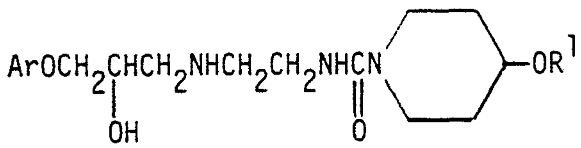

I

wherein $R^1$ is hydrogen or an acyl group

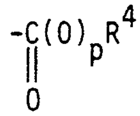

wherein p is an integer 0 or 1 and $R^4$ is an aliphatic, cycloaliphatic or aromatic group, optionally substituted, and/or interrupted by heteroatoms, and wherein Ar is a an aromatic or heteroaromatic group as known in the art.

2. A compound according to claim 1 wherein Ar is a group

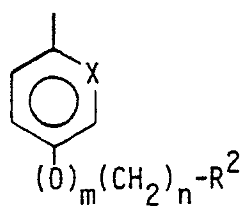

Ia

wherein m is an integer 0 or 1, n is an integer 1 to 3 provided that n is an integer 2 to 3 when m is 1 and $R^2$ is a group $-O-R^3$, $-O-(CH_2)_qO-R^3$ a pyrazole group or a group

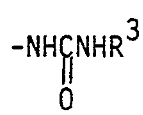

in which groups q is an integer 2 to 3 and $R^3$ is a straight or branched alkyl group having 1-8 carbon atoms or a cycloalkylalkyl group having 4-8 carbon atoms, and X is N or C-R, wherein R is H, CN or $OCH_3$, or a pharmaceutically acceptable salt of such compound.

3. A compound according to claim 2, or a pharmaceutically acceptable salt thereof, wherein X is CH.

4. A compound according to claim 3 wherein the group

$(O)_m(CH_2)_n$-$R^2$ is

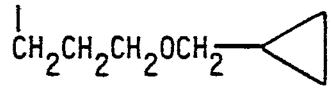

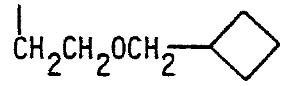

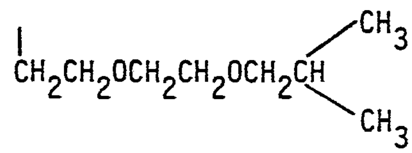

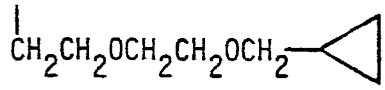

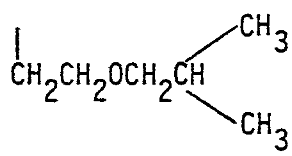

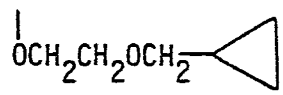

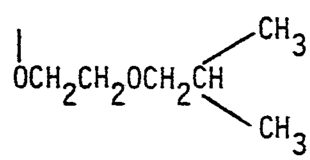

or

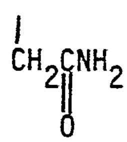

5. A compound according to one or more of the preceeding claims, or a pharmaceutically acceptable salt thereof, wherein $R^1$ is hydrogen or an acyl group

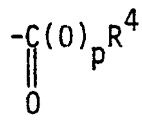

wherein p is an integer 0 or 1 and $R^4$ is a straight or branched alkyl group containing 1-20 carbon atoms, a cycloalkyl or cycloalkylalkyl group containing 3-20 carbon atoms, a phenyl, phenoxy, phenoxymethyl, benzyl or phenylethyl group, each group $R^4$ where possible optionally carrying a substituent and/or being interrupted by one or more heteroatoms.

6. A compound according to claim 5, or a pharmaceutically acceptable salt thereof, wherein $R^4$ is a straight alkyl group containing 1-12 carbon atoms, a cycloalkyl group containing 3-7 carbon atoms or a cycloalkylalkyl group containing 3-12 carbon atoms, each where possible optionally carrying a substituent and/or being interrupted by one or more heteroatoms, or a substituted phenyl, phenoxy, phenoxymethyl, benzyl or phenylethyl group having up to 12 carbon atoms.

7. A compound as defined in any of claims 1-6 in the form of a substantially pure S isomer.

8. A process for preparing a compound of the formula

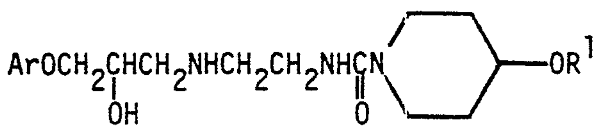

I

wherein $R^1$ is hydrogen or an acyl group

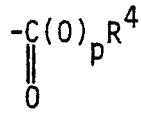

wherein p is an integer 0 or 1 and $R^4$ is an aliphatic, cycloaliphatic or aromatic group, optionally substituted or interrupted by heteroatoms, and wherein Ar is an aromatic or heteroaromatic group as known in the art and preferably a group

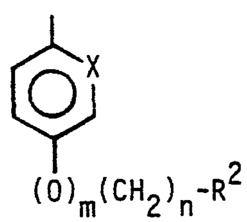

Ia

wherein m is an integer 0 or 1, n is an integer 1 to 3 provided that n is an integer 2 to 3 when m is 1, and $R^2$ is a group $O-R^3$, $-O-(CH_2)_qO-R^3$ or a pyrazole group or a group

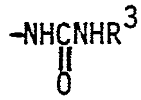

in which groups q is an integer 2 to 3 and $R^3$ is a straight or branched alkyl group having 1-8 carbon atoms or a cycloalkylalkyl group having 4-8 carbon atoms, and X is N or C-R, wherein R is H, CN or $OCH_3$ or a pharmaceutically acceptable salt of such compound, whereby below the part of formula I

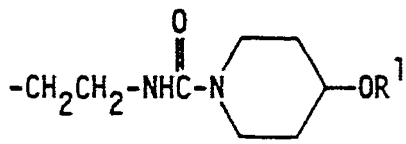

is referred to as $R^N$, characterized in

a) reacting a compound of the formula

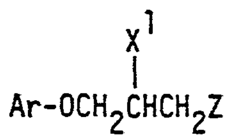

II

or

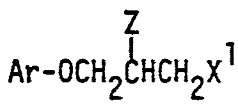

IIa

wherein

$X^1$ is a hydroxy group, Z is a hydroxy group or a reactive, esterified hydroxy group, or $X^1$ and Z together form an epoxy group, with a compound of the formula

$H_2NR^N$ III

or

b) reacting a compound of the formula

$Ar\text{-}OCH_2CHOHCH_2NH_2$ IV

wherein $R^1$ and $R^2$ are as defined above, with a compound of the formula

$ZR^N$ V

wherein Z is as defined above, or

c) reacting a compound of the formula

ArOH VI

with a compound of the formula

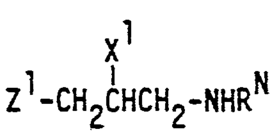

VII

wherein $X^1$ is as defined above, and $Z^1$ is Z as defined above however $Z^1$ is other than hydroxy, or

d) reacting a compound of the formula

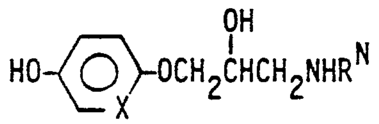

IX

with a compound of the formula

$Z^1(CH_2)_n$-$R^2$ X

wherein $Z^1$ is as defined above, to formation of a compound of formula I wherein Ar is of formula Ia and m is 1, or

e) reacting a compound of the formula

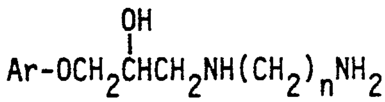

XV

wherein n is as defined above and wherein the nitrogen atom adjacent to the propanol may carry a protective group e.g. a benzyl group, with a compound of the formula

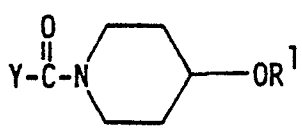

XVI

wherein $R^1$ is as defined above and Y is a leaving group such as halogen, alkoxy, an aryloxy group or an acyloxy group, or

f) splitting off a residue from a compound of formula I above further substituted with a residue, such as a benzyl or substituted benzyl group at a heteroatom, or

g) converting a compound of the formula

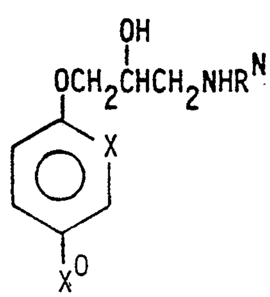

XVII

wherein X° is a group containing one or more carbon-carbon unsaturation and being convertible to the group $(O)_m(CH_2)_n-R^2$ by saturating each such unsaturation therein, or

h) reducing a Schiff's base of the formula

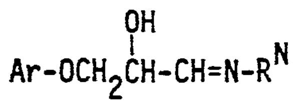

XVIII

or

i) reducing the oxo group to a hydroxy group in a compound of formula

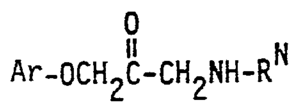

XXI

or

j) reducing an amidic carbonyl group in a compound of one of the formulas

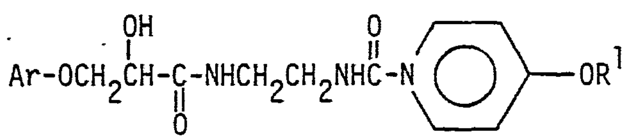

XXII

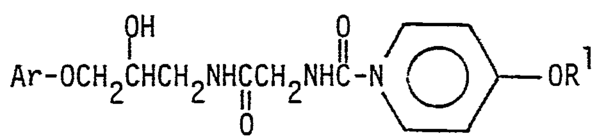

XXIII

k) to form a compound of formula I where $R^1$ is an acyl group

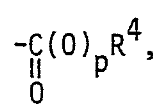

reacting the corresponding compound where $R^1$ is H with an acid or acid derivative of the formula

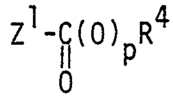

XXIV

wherein Z' is a hydroxy group or a reactive group as defined for a reactive esterified hydroxy group Z above,

I) reacting a compound of the formula

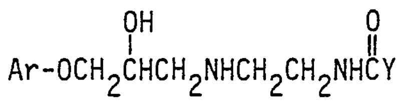

XXV

wherein the nitrogen atom adjacent to the propanol may carry a protective group, and wherein Y is a leaving group, with a compound of the formula

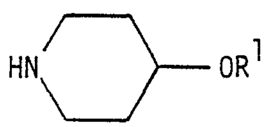

XXVI

wherein R' is as defined above,

and if desired, substituents are introduced, split off, or reacted in the compounds of the definition of the end product, or compounds obtained are transformed into other end products and/or isomer mixtures obtained are separated into pure isomers, and/or racemates obtained are separated into optical antipodes and/or free bases obtained are transformed into their pharmaceutically acceptable salt or salts obtained are transformed into their free bases.

9. A process according to claim 8 for the preparation of a compound as defined in any of claims 2-7.

10. A pharmaceutical preparation, containing a compound as defined in any of claims 1-7, or a pharmaceutically acceptable salt thereof.

11. A pharmaceutical preparation according to claim 10 in dosage unit form.

CLAIMS FOR THE CONTRACTING STATE: AT

1. A process for preparing a compound of the formula

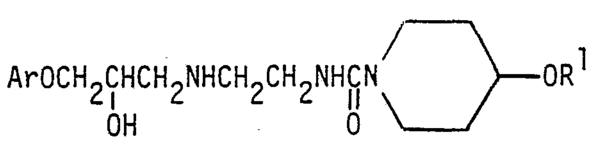

I

wherein R' is hydrogen or an acyl group

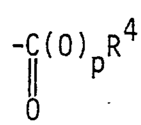

wherein p is an integer 0 or 1 and $R^4$ is an aliphatic, cycloaliphatic or aromatic group, optionally substituted or interrupted by heteroatoms, and wherein Ar is an aromatic or heteroaromatic group as known in the art and preferably a group

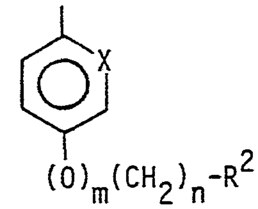

Ia

wherein m is an integer 0 or 1, n is an integer 1 to 3 provided that n is an integer 2 to 3 when m is 1, and $R^2$ is a group $O-R^3$, $-O-(CH_2)_qO-R^3$ or a pyrazole group or a group

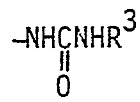

in which groups q is an integer 2 to 3 and $R^3$ is a straight or branched alkyl group having 1-8 carbon atoms or a cycloalkylalkyl group having 4-8 carbon atoms, and X is N or C-R, wherein R is H, CN or $OCH_3$ or a pharmaceutically acceptable salt of such compound, whereby below the part of formula I

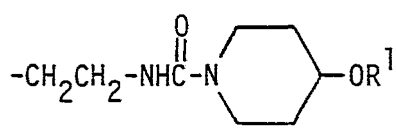

is referred to as $R^N$, characterized in

a) reacting a compound of the formula

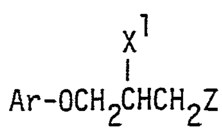

II

or

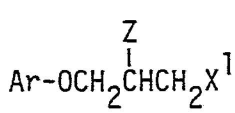

IIa

wherein

X' is a hydroxy group, Z is a hydroxy group or a reactive, esterified hydroxy group, or X' and Z together form an epoxy group, with a compound of the formula

$H_2NR^N$ III

or

b) reacting a compound of the formula

$Ar\text{-}OCH_2CHOHCH_2NH_2$ IV

wherein R' and R² are as defined above, with a compound of the formula

$ZR^N$ V

wherein Z is as defined above, or

c) reacting a compound of the formula

ArOH VI

with a compound of the formula

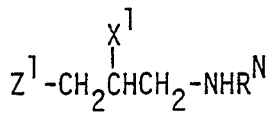

VII

wherein X' is as defined above, and Z' is Z as defined above however Z' is other than hydroxy, or

d) reacting a compound of the formula

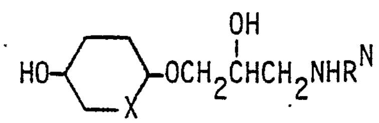

IX

with a compound of the formula

$Z'(CH_2)_n\text{-}R^2$ X

wherein Z' is as defined above, to formation of a compound of formula I wherein Ar is of formula Ia and m is 1, or

e) reacting a compound of the formula

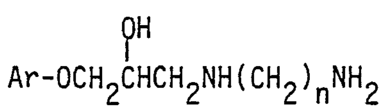

XV

wherein n is as defined above and wherein the nitrogen atom adjacent to the propanol may carry a protective group e.g. a benzyl group, with a compound of the formula

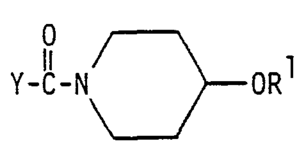

XVI

wherein $R^1$ is as defined above and Y is a leaving group such as halogen, alkoxy, an aryloxy group or an acyloxy group, or

f) splitting off a residue from a compound of formula I above further substituted with a residue, such as a benzyl or substituted benzyl group at a heteroatom, or

g) converting a compound of the formula

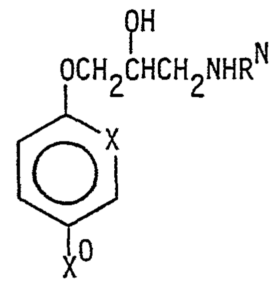

XXVII

wherein $X^0$ is a group containing one or more carbon-carbon unsaturation and being convertible to the group $(O)_m(CH_2)_n-R^2$ by saturating each such unsaturation therein, or

h) reducing a Schiff's base of the formula

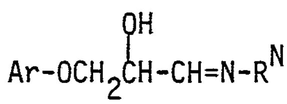

XVIII

or

i) reducing the oxo group to a hydroxy group in a compound of formula

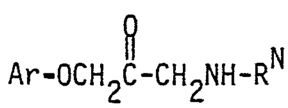

XXI

or

j) reducing an amidic carbonyl group in a compound of one of the formulas

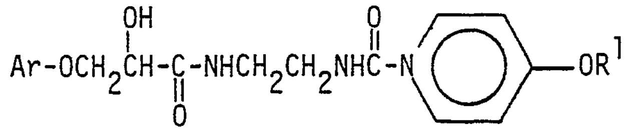

XXII

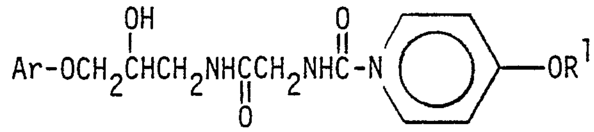

XXIII

k) to form a compound of formula I where $R^1$ is an acyl group

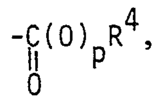

reacting the corresponding compound where $R^1$ is H with an acid or acid derivative of the formula

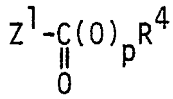

XXIV

wherein $Z^1$ is a hydroxy group or a reactive group as defined for a reactive esterified hydroxy group Z above.

l) reacting a compound of the formula

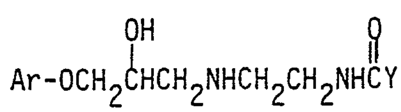

XXV

wherein the nitrogen atom adjacent to the propanol may carry a protective group, and wherein Y is a leaving group, with a compound of the formula

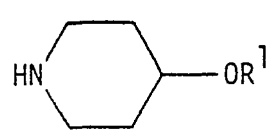

XXVI

wherein $R^1$ is as defined above.

and if desired, substituents are introduced, split off, or reacted in the compounds of the definition of the end product, or compounds obtained are transformed into other end products and/or isomer mixtures obtained are separated into pure isomers, and/or racemates obtained are separated into optical antipodes and/or free bases obtained are transformed into their pharmaceutically acceptable salt or salts obtained are transformed into their free bases.

2. A process according to claim 1 for preparing a compound of formula I wherein Ar is a group

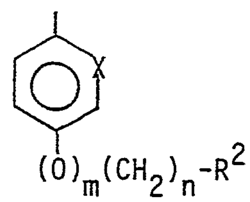

Ia

wherein m is an integer 0 or 1, n is an integer 1 to 3 provided that n is an integer 2 to 3 when m is 1 and $R^2$ is a group $-O-R^3$, $-O-(CH_2)_qO-R^3$ a pyrazole group or a group

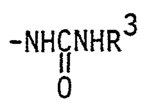

in which groups q is an integer 2 to 3 and $R^3$ is a straight or branched alkyl group having 1-8 carbon atoms or a cycloalkylalkyl group having 4-8 carbon atoms, and X is N or C-R, wherein R is H, CN or $OCH_3$, or a pharmaceutically acceptable salt of such compound.

3. A process according to claim 2 for preparing a compound of formula I, or a pharmaceutically acceptable salt thereof, wherein X is CH.

4. A process according to claim 3 for preparing a compound of formula I wherein the group

$(O)_m(CH_2)_n-R^2$ is

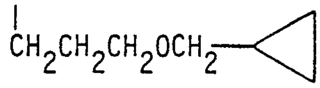

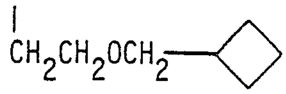

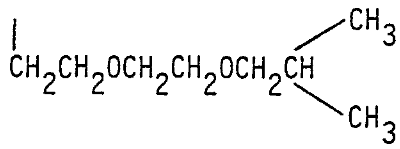

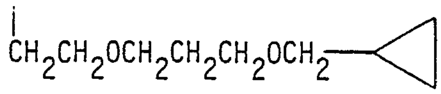

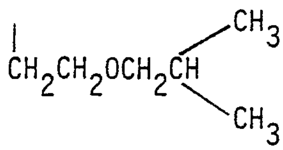

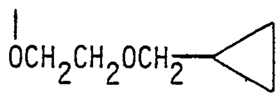

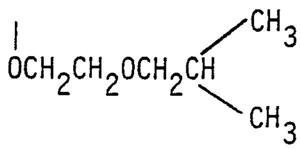

or

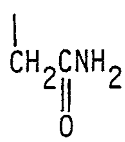

5. A process according to one or more of the preceeding claims for preparing a compound of formula I, or a pharmaceutically acceptable salt thereof, wherein R' is hydrogen or an acyl group

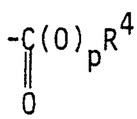

wherein p is an integer 0 or 1 and $R^4$ is a straight or branched alkyl group containing 1-20 carbon atoms, a cycloalkyl or cycloalkylalkyl group containing 3-20 carbon atoms, a phenyl, phenoxy, phenoxymethyl, benzyl or phenylethyl group, each group $R^4$ where possible optionally carrying a substituent and/or being interrupted by one or more heteroatoms.

6. A process according to claim 5 for preparing a compound of formula I, or a pharmaceutically acceptable salt thereof, wherein $R^4$ is a straight alkyl group containing 1-12 carbon atoms, a cycloalkyl group containing 3-7 carbon atoms or a cycloalkylalkyl group containing 3-12 carbon atoms, each where possible optionally carrying a substituent and/or being interrupted by one or more heteroatoms, or a substituted phenyl, phenoxy, phenoxymethyl, benzyl or phenylethyl group having up to 12 carbon atoms.

7. A process as defined in any of claims 1-6 for preparing a compound of formula I, in the form of a substantially pure S isomer.

European Patent Office

# PARTIAL EUROPEAN SEARCH REPORT

which under Rule 45 of the European Patent Convention shall be considered, for the purposes of subsequent proceedings, as the European search report

Application number
86850232.9

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | DE-A-28 22 473 (IMPERIAL INDUSTRIES LTD)<br>* page 13-14, example 2 *<br>- - - - | 1-11 | C 07 D 211/46<br>C 07 D 401/12<br>A 61 K 31/445 |
| Y | EP-A-0 052 072 (SANDOZ AG)<br>* page 35-48 *<br>- - - - | 1-11 | |
| Y | EP-A-0 097 000 (BEECHAM WUELFING) GMBH & CO KG)<br>* example 8, 10 *<br>- - - - | 1-11 | |
| Y | GB-A-1 455 116 (IMPERIAL CHEMICAL INDUSTRIES LTD)<br>* page 10-11, 20-30 *<br>- - - - | 1-11 | |

TECHNICAL FIELDS SEARCHED (Int. Cl.4)

C 07 C
C 07 D

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 2-7, 9, 11
Claims searched incompletely: 10
Claims not searched: 1, 8
Reason for the limitation of the search:

Claims 1 and 8 have not been searched because the scope of the substituent Ar is too broad.

| Place of search | Date of completion of the search | Examiner |
|---|---|---|
| STOCKHOLM | 13-08-1986 | KARLSSON G. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document